# EUROPEAN PATENT SPECIFICATION

(11) **EP 3 607 095 B1**
(45) Date of publication and mention of the grant of the patent: **25.03.2020**
(21) Application number: 19723436.2
(22) Date of filing: 16.05.2019
(51) Int. Cl.: C12Q 1/6883

(54) **KIT AND METHOD FOR DETERMINING THE RECEPTIVITY STATUS OF AN ENDOMETRIUM**
TESTSATZ UND VERFAHREN ZUR BESTIMMUNG DER EMPFÄNGLICHKEIT EINES ENDOMETRIUMS
TROUSSE ET PROCÉDÉ POUR LA DÉTERMINATION DE LA RÉCEPTIVITÉ D'UN ENDOMÈTRE

(30) Priority: 16.05.2018 EP 18382334
(43) Date of publication of application: 12.02.2020
(73) Proprietor: Integrated Genetic Lab Services SLU, 03540 Alicante (ES)
(72) Inventor: Aizpurua Sáenz, Jon, 03540 Alicante (ES); Sarasa Marcuello, Jonás, 03540 Alicante (ES); Enciso Lorences, María, 03540 Alicante (ES)
(74) Representative: Elzaburu S.L.P.
(86) International application number: PCT/EP2019/062619
(87) International publication number: WO 2019/219811

(56) References cited:
- EP-A1- 2 333 107
- EP-A1- 2 348 318
- WO-A1-2011/147976
- US-A1- 2015 038 778
- US-A1- 2018 214 068
- "Affymetrix GeneChip Human Genome U133 Array Set HG-U133A", GEO,, 11 March 2002 (2002-03-11), XP002355386,
- E.A. CAMPBELL ET AL: "Temporal expression profiling of the uterine luminal epithelium of the pseudo-pregnant mouse suggests receptivity to the fertilized egg is associated with complex transcriptional changes", HUMAN REPRODUCTION, vol. 21, no. 10, 21 June 2006 (2006-06-21) , pages 2495-2513, XP055489190, GB ISSN: 0268-1161, DOI: 10.1093/humrep/del195

SEQUENCE LISTING

<110>  Integrated Genetic Lab Services SLU

<120>  Kit and method for determining the receptivity status of an
       endometrium

<130>  PCT-924

<150>  EP18382334
<151>  2018-05-16
<160>  96

<170>  PatentIn version 3.5

<210>  1
<211>  20
<212>  DNA
<213>  Homo sapiens

<400>  1
caggagatca ggacagccta                                                   20


<210>  2
<211>  20
<212>  DNA
<213>  Homo sapiens

<400>  2
agttgccact cagttctgac                                                   20


<210>  3
<211>  22
<212>  DNA
<213>  Homo sapiens

<400>  3
   tacccctctg gacacttgga ta                                               22


<210>  4
<211>  19
<212>  DNA
<213>  Homo sapiens

<400>  4
caacaatggc cagcacaca                                                    19


<210>  5
<211>  18
<212>  DNA
<213>  Homo sapiens

<400>  5
ggtggtgctg tcgctctt                                                     18


<210>  6
<211>  22
<212>  DNA

<213>  Homo sapiens

<400>  6
gcttcccaga gtaggtgtca tt                                                22


<210>  7
<211>  20
<212>  DNA
<213>  Homo sapiens

<400>  7
agctgtgtca gatggctaca                                                   20


<210>  8
<211>  20
<212>  DNA
<213>  Homo sapiens

<400>  8
ggcatggcca ctaatggtac                                                   20


<210>  9
<211>  21
<212>  DNA
<213>  Homo sapiens

<400>  9
tgtcgatctg ctagctccct a                                                 21


<210>  10
<211>  22
<212>  DNA
<213>  Homo sapiens

<400>  10
tcagtacagt cttgccaact cc                                                22


<210>  11
<211>  20
<212>  DNA
<213>  Homo sapiens

<400>  11
tggaaaggtg gtgtggaaac                                                   20


<210>  12
<211>  19
<212>  DNA
<213>  Homo sapiens

<400>  12
gtcagctggt ggttgctaa                                                    19


<210>  13
<211>  20
<212>  DNA

<213>  Homo sapiens

<400>  13
tcaatgcaag ttcggttccc                                                    20


<210>  14
<211>  23
<212>  DNA
<213>  Homo sapiens

<400>  14
caaacccact gtttctcatc tcc                                                23


<210>  15
<211>  19
<212>  DNA
<213>  Homo sapiens

<400>  15
aagggcatcc tgctggaaa                                                     19


<210>  16
<211>  19
<212>  DNA
<213>  Homo sapiens

<400>  16
cagagatgga ggccgtgaa                                                     19


<210>  17
<211>  20
<212>  DNA
<213>  Homo sapiens

<400>  17
tggaaatccg gacgctaaca                                                    20


<210>  18
<211>  21
<212>  DNA
<213>  Homo sapiens

<400>  18
gcctttgttg cttgtgttga c                                                  21


<210>  19
<211>  20
<212>  DNA
<213>  Homo sapiens

<400>  19
cttgcctcaa tcctgcatcc                                                    20


<210>  20
<211>  18
<212>  DNA

<213>  Homo sapiens

<400>  20
agccaccagt gagcttcc                                                    18


<210>  21
<211>  21
<212>  DNA
<213>  Homo sapiens

<400>  21
acgctgagag taaaccccaa a                                                21


<210>  22
<211>  22
<212>  DNA
<213>  Homo sapiens

<400>  22
caaacttgct tcccgttctt ca                                               22


<210>  23
<211>  19
<212>  DNA
<213>  Homo sapiens

<400>  23
tgtggggtag ccatggaaa                                                   19


<210>  24
<211>  18
<212>  DNA
<213>  Homo sapiens

<400>  24
gggtcgcatg ttgtggaa                                                    18


<210>  25
<211>  20
<212>  DNA
<213>  Homo sapiens

<400>  25
tttgtcaacg tggccagcta                                                  20


<210>  26
<211>  21
<212>  DNA
<213>  Homo sapiens

<400>  26
aaagcccaga atgaccagac c                                                21


<210>  27
<211>  20
<212>  DNA

<213>  Homo sapiens

<400>  27
tctcctgccg acaagaccaa                                                    20


<210>  28
<211>  19
<212>  DNA
<213>  Homo sapiens

<400>  28
gtggtgggga aggacagga                                                     19


<210>  29
<211>  20
<212>  DNA
<213>  Homo sapiens

<400>  29
gcaagaaggt gctgacttcc                                                    20


<210>  30
<211>  20
<212>  DNA
<213>  Homo sapiens

<400>  30
gcttgtcaca gtgcagttca                                                    20


<210>  31
<211>  19
<212>  DNA
<213>  Homo sapiens

<400>  31
agagtgattc gcgtgggta                                                     19


<210>  32
<211>  21
<212>  DNA
<213>  Homo sapiens

<400>  32
ctttcaatgt tgccaccaca c                                                  21


<210>  33
<211>  20
<212>  DNA
<213>  Homo sapiens

<400>  33
ttccggatct acccctggat                                                    20


<210>  34
<211>  19
<212>  DNA

<213>  Homo sapiens

<400>  34
tctggtagcg ggtgtaggt                                                    19


<210>  35
<211>  23
<212>  DNA
<213>  Homo sapiens

<400>  35
gggcaagcaa tatcctatta ccc                                               23


<210>  36
<211>  20
<212>  DNA
<213>  Homo sapiens

<400>  36
tagctgggct cacaatcaca                                                   20


<210>  37
<211>  21
<212>  DNA
<213>  Homo sapiens

<400>  37
cagctgatcc gattcctgaa a                                                 21


<210>  38
<211>  20
<212>  DNA
<213>  Homo sapiens

<400>  38
gttggcttcc ttcacaggac                                                   20


<210>  39
<211>  24
<212>  DNA
<213>  Homo sapiens

<400>  39
actctgagga ttcctgttcc tgta                                              24


<210>  40
<211>  22
<212>  DNA
<213>  Homo sapiens

<400>  40
ccagtgtgcc tattccctga aa                                                22


<210>  41
<211>  23
<212>  DNA

<213>  Homo sapiens

<400>  41
gaatgtatac aagcagggcc aaa                                              23


<210>  42
<211>  20
<212>  DNA
<213>  Homo sapiens

<400>  42
cgtgcagaag taggcattcc                                                  20


<210>  43
<211>  20
<212>  DNA
<213>  Homo sapiens

<400>  43
aaatgcagcg tgggatttcc                                                  20


<210>  44
<211>  21
<212>  DNA
<213>  Homo sapiens

<400>  44
ccagacaggt ggcttgtatc a                                                21


<210>  45
<211>  19
<212>  DNA
<213>  Homo sapiens

<400>  45
atggtctcgg gaaggtgac                                                   19


<210>  46
<211>  21
<212>  DNA
<213>  Homo sapiens

<400>  46
ggtgatttct accgctggaa c                                                21


<210>  47
<211>  20
<212>  DNA
<213>  Homo sapiens

<400>  47
ttggtacagg gctccagaaa                                                  20


<210>  48
<211>  19
<212>  DNA

<213>  Homo sapiens

<400>  48
tctgccagaa tgcagccta                                                    19


<210>  49
<211>  18
<212>  DNA
<213>  Homo sapiens

<400>  49
agtggcacca ccacaaca                                                     18


<210>  50
<211>  19
<212>  DNA
<213>  Homo sapiens

<400>  50
gcaaaggcgt cgtcaatca                                                    19


<210>  51
<211>  19
<212>  DNA
<213>  Homo sapiens

<400>  51
tcctcggctt gcaatggsa                                                    19


<210>  52
<211>  20
<212>  DNA
<213>  Homo sapiens

<400>  52
ctttgcactt gcaggaacca                                                   20


<210>  53
<211>  18
<212>  DNA
<213>  Homo sapiens

<400>  53
gctcctgcaa gtgcaaaa                                                     18


<210>  54
<211>  19
<212>  DNA
<213>  Homo sapiens

<400>  54
gcagctgcac ttctctgac                                                    19


<210>  55
<211>  19
<212>  DNA

<213>  Homo sapiens

<400>  55
cagctcctgc aagtgcaaa                                                    19


<210>  56
<211>  17
<212>  DNA
<213>  Homo sapiens

<400>  56
gacgcccctt tgcagac                                                      17


<210>  57
<211>  20
<212>  DNA
<213>  Homo sapiens

<400>  57
cctcgaaatg gaccccaact                                                   20


<210>  58
<211>  21
<212>  DNA
<213>  Homo sapiens

<400>  58
gcactctttg catttgcagg a                                                 21


<210>  59
<211>  20
<212>  DNA
<213>  Homo sapiens

<400>  59
cacctccact ccatcctgaa                                                   20


<210>  60
<211>  22
<212>  DNA
<213>  Homo sapiens

<400>  60
ggtagccatg gatagaggct aa                                                22


<210>  61
<211>  22
<212>  DNA
<213>  Homo sapiens

<400>  61
agccaagaag agttcctctg ta                                                22


<210>  62
<211>  20
<212>  DNA

<213>  Homo sapiens

<400>  62
ttgacttcgt agcccttcca                                                    20


<210>  63
<211>  20
<212>  DNA
<213>  Homo sapiens

<400>  63
ggcaaggtgt tcgatgtgac                                                    20


<210>  64
<211>  22
<212>  DNA
<213>  Homo sapiens

<400>  64
ctcttccagc aaagacccca ta                                                 22


<210>  65
<211>  19
<212>  DNA
<213>  Homo sapiens

<400>  65
aggctccaca atggaggaa                                                     19


<210>  66
<211>  21
<212>  DNA
<213>  Homo sapiens

<400>  66
tcatcatcac tgtccgagct a                                                  21


<210>  67
<211>  21
<212>  DNA
<213>  Homo sapiens

<400>  67
gcggctcttt aaggatggga a                                                  21


<210>  68
<211>  20
<212>  DNA
<213>  Homo sapiens

<400>  68
aacacaggcg cctcttctac                                                    20


<210>  69
<211>  20
<212>  DNA

<213>  Homo sapiens

<400>  69
gtgcccacag tgtttgagaa                                                   20


<210>  70
<211>  19
<212>  DNA
<213>  Homo sapiens

<400>  70
tgtgtcccac aaagccaac                                                    19


<210>  71
<211>  18
<212>  DNA
<213>  Homo sapiens

<400>  71
gaggccccta ccacttcc                                                     18


<210>  72
<211>  20
<212>  DNA
<213>  Homo sapiens

<400>  72
gccgtcaaac accttgagac                                                   20


<210>  73
<211>  18
<212>  DNA
<213>  Homo sapiens

<400>  73
ctctcccagc actgctac                                                     18


<210>  74
<211>  23
<212>  DNA
<213>  Homo sapiens

<400>  74
cttgtggatt gattgtcttg gaa                                               23


<210>  75
<211>  19
<212>  DNA
<213>  Homo sapiens

<400>  75
acatcggaac tgcgactca                                                    19


<210>  76
<211>  19
<212>  DNA

<213>  Homo sapiens

<400>  76
ttcttgcaac acgcttccc                                                    19


<210>  77
<211>  23
<212>  DNA
<213>  Homo sapiens

<400>  77
ccacgatatc atcaccaagt tcc                                               23


<210>  78
<211>  20
<212>  DNA
<213>  Homo sapiens

<400>  78
ttgacccagg acgctcttca                                                   20


<210>  79
<211>  17
<212>  DNA
<213>  Homo sapiens

<400>  79
ggccttgctg tcctcca                                                      17


<210>  80
<211>  17
<212>  DNA
<213>  Homo sapiens

<400>  80
cccggttgtt gcactcc                                                      17


<210>  81
<211>  19
<212>  DNA
<213>  Homo sapiens

<400>  81
ccaaccgcga gaagatgac                                                    19


<210>  82
<211>  20
<212>  DNA
<213>  Homo sapiens

<400>  82
tagcacagcc tggatagcaa                                                   20


<210>  83
<211>  19
<212>  DNA

<213>  Homo sapiens

<400>  83
ttagctgtgc tcgcgctac                                                    19


<210>  84
<211>  22
<212>  DNA
<213>  Homo sapiens

<400>  84
ctctgctgga tgacgtgagt aa                                                22


<210>  85
<211>  19
<212>  DNA
<213>  Homo sapiens

<400>  85
ccatggcccc tcccatcta                                                    19


<210>  86
<211>  20
<212>  DNA
<213>  Homo sapiens

<400>  86
ggtgcacaca tccttggcta                                                   20


<210>  87
<211>  20
<212>  DNA
<213>  Homo sapiens

<400>  87
cagacccgaa ttcccagaac                                                   20


<210>  88
<211>  22
<212>  DNA
<213>  Homo sapiens

<400>  88
ctcgaacact gagcttgtgt aa                                                22


<210>  89
<211>  21
<212>  DNA
<213>  Homo sapiens

<400>  89
gaacgggaag cttgtcatca a                                                 21


<210>  90
<211>  20
<212>  DNA

<213>  Homo sapiens

<400>  90
atcgccccac ttgattttgg                                                    20


<210>  91
<211>  20
<212>  DNA
<213>  Homo sapiens

<400>  91
tgcccgaaac gccgaatata                                                    20


<210>  92
<211>  20
<212>  DNA
<213>  Homo sapiens

<400>  92
cgtggttcgt ggctctctta                                                    20


<210>  93
<211>  19
<212>  DNA
<213>  Homo sapiens

<400>  93
acgctacagc agcagctaa                                                     19


<210>  94
<211>  19
<212>  DNA
<213>  Homo sapiens

<400>  94
agctcgattg gcacggtta                                                     19


<210>  95
<211>  21
<212>  DNA
<213>  Homo sapiens

<400>  95
agacagcacg ctaataatgc a                                                  21


<210>  96
<211>  19
<212>  DNA
<213>  Homo sapiens

<400>  96
agcttcgtct ccttgggta                                                     19

## Description

### FIELD OF THE INVENTION

The present invention belongs to the field of reproductive medicine. The present invention relates to a kit for determining the receptivity status of an endometrium comprising means for quantifying the expression of several genes as well as to a method for determining the receptivity status of an endometrium comprising the quantification of the expression of said genes.

### BACKGROUND OF THE INVENTION

One of the key processes for the establishment of a successful pregnancy is embryonic implantation into the endometrium. Implantation is a complex process that involves an intricate dialog between the embryo and the endometrial cells (Singh et al., 2011). This interaction is essential for the apposition, adhesion and invasion of the blastocyst in the human endometrium (Giudice and Irwin, 1999).

The human endometrium is a highly dynamic structure, which undergoes periodical changes during the menstrual cycle in order to reach a receptive status adequate for embryonic implantation. This period of receptivity is known as the window of implantation (WOI) and occurs between Days 19 and 21 of the menstrual cycle (Navot et al., 1991; Harper, 1992). In any other phase of the menstrual cycle, the endometrium is not receptive to pregnancy (Garrido-Gómez et al., 2013). Successful implantation requires therefore a viable embryo and synchrony between it and the receptive endometrium (Teh et al., 2016). The correct identification and prediction of the period of uterine receptivity is essential to maximize the effectiveness of assisted reproduction treatments.

The study of endometrial receptivity is not new as histological analysis has been traditionally used for endometrial dating (Noyes et al. 1950); however, the accuracy of this method to predict endometrial receptivity has been shown to be limited (Coutifaris et al., 2004; Murray et al., 2004). Some alternative methods to evaluate endometrial receptivity have been developed in the last decade: biochemical markers (Zhang et al., 2012), soluble ligands (Thouas et al., 2015), hormone receptors (Aghajanova et al., 2009), cytokines (Paiva et al., 2011), microRNAs (Sha et al., 2011) or HOX-class homeobox genes (Kwon and Taylor, 2004).

Other studies, focused on the understanding of the molecular mechanisms underlying the histological changes of the endometrium during the menstrual cycle, have identified specific genes responsible for the alterations observed (Talbi et al., 2006). Some other reports have addressed this molecular analysis from a wider perspective, performing a global screening of the transcriptome at different moments of the menstrual cycle (Carson, 2002; Ponnampalam et al., 2004), or under different infertility conditions (Koler et al., 2009; Altmäe et al., 2010), pathologies (Kao et al., 2003) or ovarian stimulation protocols (Horcajadas et al., 2005). Valuable information about the process of endometrial proliferation can be extracted from these studies. However, even though the list of studies is long, the number of molecular diagnostic tools to identify the moment of uterine receptivity is short (Lessey et al., 1995 Dubowy et al., 2003; Díaz-Gimeno et al., 2011).

WO2011147976 A1 discloses a method for assessing the endometrium receptivity of a patient by measuring the expression level of eleven genes MFAP5, ANGPTLI, PROKI, NLF2, LAMB3, BCL2L10, CD68, TRPC4, SORCSI, FST and KRT80 in an endometrial biopsy sample obtained from said patient.

US2018214068 A1 published on 02-08-2018, discloses a method and kit for determining the receptivity status of an endometrium for embryonic implantation based on the determination of the expression of sets of genes, e.g. a set comprising ANXA4, CATSPERB, PTGFR, PTGS1, IL8, SCGB2A2, ANGPTL1, HPRT1, MMP10, PGR, ITGA8, IFNG, PROK1, FOX01, CXCL1, STC1, MMP9, MUC1, RPL13A, CALCA, ITGA9, RACGAP1, GPX3, PPP2R2C, ARG2, SCGB3A1, ALDH1A3, APOD, C2CD4B, TFF3, AQP3, GJA4, ARHGDIA, SELL, APOL2, MT1H, MT1X, MT1L, MAOA and MT1F.

Therefore, there is a need for more accurate and easy to use diagnostic tools to assess the receptivity status of an endometrium and to maximize the effectiveness of assisted reproduction treatments.

### SUMMARY

In a first aspect, the present invention relates to kit for determining the receptivity status of an endometrium comprising means for quantifying the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, wherein said kit comprises means for quantifying the expression of up to 100 genes, preferably up to 48 genes.

### DESCRIPTION

The identification of the optimal time for embryo transfer is essential to maximize the effectiveness of assisted reproduction treatment. The inventors have surprisingly found a highly reliable test for endometrial receptivity, capable of determining if the status of an endometrium is receptive or non-receptive. Moreover, in the case of a non-receptive endometrium, the test developed by the inventors is capable of distinguishing between a pre-receptive and post-receptive endometrium.

The present invention provides a very useful tool for clinical practice routine to help guide embryo transfers to be performed at the best endometrial moment, guaranteeing embryo-endometrial synchrony and thus, allowing for the achievement of better assisted reproduction treatment results. Couples with repeated implantation failure or previously failed in vitro fertilization (IVF) cycles or couples with recurrent miscarriage can benefit from the detailed analysis of endometrial receptivity and embryo-endometrial synchronization.

The present invention is a new step in the field of personalized medicine in human reproduction in the management of the endometrium in preparation for embryo transfer, with the final goal of achieving better assisted reproduction treatment results, increasing embryo implantation rates and the likelihood of successful pregnancies.

In a preferred embodiment of the present invention, the kit of the first aspect further comprises means for quantifying the expression of at least one of the following genes: ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

In a preferred embodiment of the present invention, the kit of the first aspect comprises means for quantifying the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

In a preferred embodiment of the present invention, the kit of the first aspect comprises means for quantifying the expression of substantially only the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3. As used herein, the expression "substantially only" means that if any other gene is quantified, then said other gene or genes is or are reference genes, which are only quantified for serving as a reference for the quantification of the expression, as a reference for the sample load.

In a preferred embodiment of the present invention the means for quantifying the genes comprise a pair of primers for each one of the genes.

In a preferred embodiment of the present invention, the kit of the first aspect further comprises a pair of primers for at least one reference gene. Preferably, the reference gene or genes is or are selected from actin, beta-2 microglobulin, cytochrome C1, EMG1 N1-specific pseudouridine methyltransferase, glyceraldehyde-3-phosphate dehydrogenase, TATA-box binding protein, topoisomerase (DNA) I, tyrosine 3-monooxygenase/tryptophan 5-monooxygenase activation protein zeta and combinations thereof. In a preferred embodiment, the kit of the first aspect comprises means for quantifying the expression of all the eight reference genes mentioned above. In another preferred embodiment, the reference genes are cytochrome C1, EMG1 N1-specific pseudouridine methyltransferase and glyceraldehyde-3-phosphate dehydrogenase (CYC1, EMG1, GAPDH).

Therefore, in a preferred embodiment, the kit of the invention consists essentially of means for quantifying the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3, actin, beta-2 microglobulin, cytochrome C1, EMG1 N1-specific pseudouridine methyltransferase, glyceraldehyde-3-phosphate dehydrogenase, TATA-box binding protein, topoisomerase (DNA) I and tyrosine 3-monooxygenase/tryptophan 5-monooxygenase activation protein zeta. As used herein, the expression "consists essentially of" means that no other genes are quantified and that the kit may contain other reagents such as buffers, polymerase enzymes, nucleotides mixes, etc, which are necessary for the analysis of the expression of the above mentioned genes.

In a preferred embodiment of the present invention, the kit of the first aspect further comprises a master mix comprising a DNA-binding dye.

In another preferred embodiment of the present invention, the kit of the first aspect further comprises a specific probe for each one of the genes. Preferably the probe is labeled, preferably dual-labeled. As used herein, the term "labeled" means that the probe is marked by means of a fluorescent reporter dye on the 5' base and a quencher located on the 3' base.

In a preferred embodiment of the present invention, the kit of the first aspect further comprises at least one of the following: a DNA polymerase, a nucleotide mix, a buffer, DNase-free water.

In a second aspect, the present invention relates to a method for determining the receptivity status of an endometrium comprising the quantification of the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, in a sample.

In a preferred embodiment of the second aspect, the method further comprises the quantification of the expression of at least one of the following genes: ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

In a more preferred embodiment of the second aspect of the invention, the expression of the following genes is quantified: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

In a preferred embodiment of the present invention, the method of the second aspect further comprises quantifying the expression of at least one reference gene in the sample. Preferably, the reference gene or genes is or are selected from actin, beta-2 microglobulin, cytochrome C1, EMG1 N1-specific pseudouridine methyltransferase, glyceraldehyde-3-phosphate dehydrogenase, TATA-box binding protein, topoisomerase (DNA) I, tyrosine 3-monooxygenase/tryptophan 5-monooxygenase activation protein zeta and combinations thereof. In another preferred embodiment, the reference genes are cytochrome C1, EMG1 N1-specific pseudouridine methyltransferase and glyceraldehyde-3-phosphate dehydrogenase (CYC1, EMG1, GAPDH).

In a preferred embodiment of the second aspect of the invention, the quantification is made by qPCR, preferably by RT-qPCR.

In a more preferred embodiment of the second aspect of the invention, the sample is a cDNA sample, preferably a cDNA obtained from an RNA sample from a female subject, preferably a female human. More preferably, the RNA has been obtained from an endometrial biopsy.

In a preferred embodiment of the method of the invention, the results of the gene expression levels can classify an endometrium into: "receptive" or "non receptive". When an endometrium is classified as "receptive", it means that the window of implantation matches the day on which the biopsy was taken. The embryo transfer should be scheduled to be performed on the same day and type of cycle on which the biopsy was taken. If an endometrium is classified as "non-receptive" (either pre-receptive or post-receptive), it means that the WOI is displaced. A new endometrial biopsy on the day estimated needs to be obtained so that the WOI is identified and a personalized embryo transfer is scheduled.

Another aspect of the present disclosure is a method for improving the pregnancy success of an IVF treatment comprising:
(i) determining the receptivity status of an endometrium following the method of the second aspect of the present invention,
(ii) determining the day in which the endometrium is receptive following the method of the present invention, and
(iii) transferring at least one embryo, preferably at blastocyst stage, at the day determined in step (ii).

The present invention relates to a method for determining if the status of an endometrium is receptive or non-receptive, and in case it is non-receptive, if it is pre-receptive or post-receptive. The method comprises analyzing in a sample obtained from an endometrium, preferably a human endometrium, the gene expression levels of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

The mRNA of an endometrial biopsy is extracted and purified by methods known in the art. The expression levels of the above mentioned genes are determined and said expression levels are analyzed by a computer software comprising a specific prediction model which classifies and determines the status of the endometrium depending on the gene expression levels. The prediction model is a mathematical system using different algorithms, formulas, to distinguish between a receptive profile, a pre-receptive profile and a post-receptive profile.

The prediction model is designed from the data obtained from the analysis of the expression of the above mentioned genes in different samples, as explained in the examples "Patient selection and sample collection" and "Principal component analysis and discriminant functional analysis" sections.

### EXAMPLES

### RNA extraction and cDNA preparation

Total RNA was extracted using RNeasy mini kit (Qiagen, London, UK); RNA purity and concentration was confirmed by NanoDrop 2000 Spectophotometer (Thermo Scientific, Waltham, MA, USA) and RNA integrity was assessed using Agilent Bioanalyzer 2100 (Agilent Technologies, Santa Clara, CA, USA) following the manufacturer's instructions. RNAs were reverse-transcribed into cDNA using Fluidigm Reverse Transcription Master Mix (Fluidigm, San Francisco, CA, USA) and either immediately used or stored at -20°C until further downstream processing.

### Gene expression analysis

The pairs of primers targeting the selected and reference genes were designed and are indicated in the sequence listing. Table 2 includes the primer sequences.

Specific target amplification (STA) was carried out on cDNA samples using Fluidigm PreAmp Master Mix and DELTAgene assays following the manufacturer's instructions (Fluidigm, San Francisco, CA). RT-qPCR reactions were performed following the Fast Gene Expression Analysis Using Evagreen on the Biomark HD Systemand 96.96 Dynamic Array™ IFC (Fluidigm, San Francisco, CA).

Data was collected with Fluidigm® Real-Time PCR analysis software using linear baseline correction method and global auto Cq threshold method. Data were then exported to Excel as.csv files and Cq values were normalized using the mean of the reference genes selected.

### Study design

Gene expression data from endometrial biopsies obtained at different moments of the menstrual cycle from healthy fertile donors (Group A) and subfertile women (Group B) were analyzed.

### Patient selection and sample collection

Group A consisted of 96 healthy fertile donors (18-34 years), with regular menstrual cycles and normal BMI (25-30). Endometrial biopsies from this group were obtained on two different days of the same natural menstrual cycle: LH + 2, i.e. 2 days after the luteinizing hormone surge and LH + 7, i.e. 7 days after the LH surge. Group B consisted of 120 subfertile patients (30-42 years) undergoing hormone-replacement therapy (HRT) cycles. Endometrial biopsies from this group of patients were obtained after five full days of progesterone administration (P₄+5). Endometrial biopsies of approximately 30 mg were obtained from the uterine fundus using a Pipelle catheter (Gynetics, Namont-Achel, Belgium) under sterile conditions. Tissue was then placed in a CryoTube® (Nunc, Roskilde, Denmark) containing 1 ml RNAlater® (Sigma-Aldrich, St Louis, MO, USA) and stored at -20 °C until further processing.

### Principal component analysis and discriminant functional analysis

Differential expression of genes from LH + 2 and LH + 7 groups was assessed by comparing ΔCq values (paired t-test (P < 0.05)). Fold change (-ΔΔCq) was calculated to determine upregulated and downregulated genes in the WOI. In order to assess if receptivity status could be established with a reduced number of genes, a principal component analysis (PCA) of the genes showing significant fold change between LH + 2 and LH + 7 was performed. Discriminant functional analysis (DA) was then used to evaluate the ability of the genes with the highest absolute coefficient value from each of the leading principal components to accurately discriminate samples into the following states: proliferative, receptive, pre-receptive and post-receptive. A Split- Sample validation of the DA was performed to assess the reliability and robustness of discriminant findings. Both fertile and infertile patient samples were split into two subsets. One data set (70% of the samples) was used as a training set and the other one as testing set (remaining 30% of the samples). The percentage of correct classifications was calculated to determine the reliability of the DA model. Data analyses were performed by using IBM SPSS Statistics software version 19.0.

### Clinical experience: reproductive outcomes

Analysis of the outcomes after the first embryo transfer (blastocyst) when using the method of the invention indicated a significantly higher positive pregnancy rate (gestational sac at 8 weeks and heartbeat positive at 12 weeks) in the group of patients that followed the progesterone pretreatment recommendation using the method of the invention, compared to those that deviated more than 12 h from this recommendation.

In total, 741 cases were analysed, with an average of 1.16 embryos transferred and a mean age of the women of 41.41 years for the group that followed the progesterone pretreatment recommendation using the method of the invention, and an average of 1.28 embryos transferred and a mean age of the women of 41.06 years for the group that deviated more than 12 h from this recommendation.

Within the group of cases that followed the recommendation using the method of the present invention, clinical pregnancies were almost two times higher than the ones from the group whose transfers deviated more than 12 h from the recommendation (that is, that deviated more than 12 h from the WOI). These differences were statistically significant (44.5% vs 26%, p=0.002, n=741).

Pregnancy rates of the group of transfers performed with a deviation of more than 12 h were studied in further detail. This group was divided into:
(i) transfers performed between 12 and 24 h from the recommendation using the method of the invention (WOI), and
(ii) transfers performed with more than 24 h deviation from this window.

For the group that deviated from 12 to 24 h from the recommendation, an average of 1.29 embryos were transferred and the mean age of the women was 41.40 years. For the group that deviated from more than 24 h from the recommendation, an average of 1.27 embryos were transferred and the mean age of the women was 40.62 years.

The group were the transfer was performed with more than 24 h of deviation from the WOI showed a significant decline in pregnancy rates. The pregnancy rates of the three groups were the following:
- following the method of the invention: 44.5 %
- from 12 to 24 h deviation: 33.3 %
- more than 24 h deviation: 15.6 %

These differences in the pregnancy rates of the three groups were significant (p=0.001).

Within the group of cases that followed the recommendation using the method of the present invention, clinical pregnancies were almost four times higher than the ones from the group whose transfers deviated more than 24 h from the recommendation.

### Successful pregnancies after following the embryonic transfer recommendations of the test of the invention

Endometrial biopsies from 11 women were obtained after four to six full days of progesterone administration (P₄+ 6). All the women were in a hormone replacement therapy cycle (HRT).

The receptivity of the endometrium was analysed using the method of the invention and the results of the following table were obtained.

When the endometrium was found to be Receptive, it was recommended to transfer the embryo (at blastocyst stage) after the same number of days of progesterone administration as when the biopsy was obtained.

**Table 1. Examples of successful pregnancies after using the method of the invention.**

| | | | | | | | Pregnancy rate | | | |
|---|---|---|---|---|---|---|---|---|---|---|
| Sample | Type of cycle | Biopsy at P₄+ | Result of the test of the invention | Transfer reccommended at P₄+ | Transfer at P₄+ | Embryonic development (day) | β-hCG positive | Sac positive | Heartbeat positive | Delivery positive |
| 1 | HRT | 5 | Receptive | 5 | 5 | 5 | 1 | 1 | 1 | 1 |
| 2 | HRT | 5 | Pre-receptive | 6 | 6 | 5 | 1 | 1 | 1 | 1 |
| 3 | HRT | 4,5 | Receptive | 4,5 | 4,5 | 5 | 1 | 1 | 1 | 1 |
| 4 | HRT | 5,5 | Receptive | 5,5 | 5,5 | 5 | 1 | 1 | 1 | 1 |
| 5 | HRT | 5 | Pre-receptive | 6 | 6 | 5 | 1 | 1 | 1 | 1 |
| 6 | HRT | 4 | Pre-receptive | 4,5 | 4,5 | 5 | 1 | 1 | 1 | 1 |
| 7 | HRT | 5 | Receptive | 5 | 5 | 5 | 1 | 1 | 1 | 1 |
| 8 | HRT | 5 | Post-receptive | 4,5 | 4,5 | 5 | 1 | 1 | 1 | 1 |
| 9 | HRT | 6 | Post-receptive | 5 | 5 | 5 | 1 | 1 | 1 | 1 |
| 10 | HRT | 5 | Pre-receptive | 6 | 6 | 5 | 1 | 1 | 1 | 1 |
| 11 | HRT | 5,5 | Post-receptive | 4 | 4 | 5 | 1 | 1 | 1 | 1 |

| | | | | | | | | | | |
|---|---|---|---|---|---|---|---|---|---|---|
| HRT: hormone-replacement therapy (HRT) cycle P₄+5: obtained after five full days of progesterone impregnation β-hCG positive result >25 IU/I; Sac and heartbeat positive determined by ultrasound at 8 and 12 weeks respectively. | | | | | | | | | | |

When the endometrium was found to be Pre-Receptive, it was recommended to transfer the embryo (at blastocyst stage) after one day or half a day more in respect to the number of days of progesterone administration when the biopsy was obtained.

When the endometrium was found to be Post-Receptive, it was recommended to transfer the embryo (at blastocyst stage) after one day or half a day less in respect to the number of days of progesterone administration when the biopsy was obtained.

Following this strategy, pregnancy and healthy births were achieved in all 11 cases (table 1).

### Clinical case reports

### Case 1:

A woman with a history of 2 gestations, 2 miscarriages and no deliveries had received 5 IVF treatments without any pregnancy outcome and 5 IVF after preimplantation genetic testing for aneuploidy (PGT-A), without any pregnancy either.

The method of the invention was performed on an endometrial biopsy from this woman at day P₄+7 (7 full days of progesterone administration) and was determined to be receptive. It was recommended to transfer the embryos at day P₄+7. Following this recommendation, pregnancy was achieved (positive β-hGC (beta human chorionic gonadotropin) and positive heartbeat).

### Case 2:

A woman with a history of no gestations, no miscarriages and no deliveries had received 4 artificial insemination treatments, 9 IVF+PGT-A treatments and 1 oocyte donation without any pregnancy outcome.

The method of the invention was performed on an endometrial biopsy from this woman at day P₄+5.5 (5.5 days of progesterone administration) and was determined to be Pre-receptive. It was recommended to transfer the embryos at day P₄+6.5. Following this recommendation, pregnancy was achieved (positive β-hGC (beta human chorionic gonadotropin), heartbeat and birth).

### Case 3:

A woman with a history of 5 gestations, 5 miscarriages and no deliveries had received 3 artificial insemination treatments followed by 2 miscarriages, 1 IVF+PGT-A treatment followed by miscarriage and 1 oocyte donation + PGT-A followed by 2 cryotransfers and 2 biochemical miscarriages.

The method of the invention was performed on an endometrial biopsy from this woman at day P₄+5.5 (5.5 days of progesterone administration) and was determined to be Pre-receptive. It was recommended to transfer the embryos at day P₄+7. Following this recommendation, pregnancy was achieved (positive β-hGC (beta human chorionic gonadotropin) and positive heartbeat).

### REFERENCES

Aghajanova L et al. Fertil Steril 2009;91:2602-2610.
Altmäe S et al. Mol Hum Reprod 2010;16:178-187.
Carson DD. Mol Hum Reprod 2002;8:871-879.
Coutifaris C et al. Fertil Steril 2004;82:1264-1272.
Dubowy RL et al. Fertil Steril 2003;80:146-156.
Díaz-Gimeno P et al. Fertil Steril 2011;95: 50-60. 60-15.
Garrido-Gómez T et al. Fertil Steril 2013;99:1078-1085.
Giudice LC, Irwin JC. Semin Reprod Endocrinol 1999;17:13-21.
Harper MJK. Baillieres Clin Obstet Gynaecol 1992;6:351-371.
Horcajadas JA et al. Mol Hum Reprod 2005;11:195-205.
Kao LC et al. Endocrinology 2003;144:2870-2881.
Koler M et al. Hum Reprod 2009;24:2541-2548.
Kwon HE, Taylor HS. Ann N Y Acad Sci 2004;1034:1-18.
Lessey BA et al. Fertil Steril 1995;63:535-542.
Murray MJ et al. Fertil Steril 2004;81:1333-1343.
Navot D et al. Fertil Steril 1991;55:114-118.
Noyes RW, Hertig AT, Rock J. Fertil Steril 1950;1:561-564.
Paiva P et al. Hum Reprod 2011;26:1153-1162.
Ponnampalam AP et al. Mol Hum Reprod 2004;10:879-893.
Sha AG et al. Fertil Steril 2011;96:150-155.e5.
Singh M J Endocrinol 2011;210:5-14.
Talbi S et al. 2006;147:1097-1121.
Teh WT et al. J Assist Reprod Genet 2016;33:1419-1430.
Thouas GA et al. Endocr Rev 2015;36:92-130.
Zhang D et al. Reprod Biol Endocrinol 2012;10:106.

**Table 2. Primer sequences.**

| **GENE** | **PRIMER** | **SEQ ID NO:** | **SEQUENCE** | **Gene Full Name** |
|---|---|---|---|---|
| ANXA4 | forward (F) | 1 | CAGGAGATCAGGACAGCCTA | annexin A4 |
| | reverse (R) | 2 | AGTTGCCACTCAGTTCTGAC | |
| AQP3 | F | 3 | TACCCCTCTGGACACTTGGATA | aquaporin 3 (Gill blood group) |
| | R | 4 | CAACAATGGCCAGCACACA | |
| AREG | F | 5 | GGTGGTGCTGTCGCTCTT | amphiregulin |
| | R | 6 | GCTTCCCAGAGTAGGTGTCATT | |
| ARG2 | F | 7 | AGCTGTGTCAGATGGCTACA | arginase 2 |
| | R | 8 | GGCATGGCCACTAATGGTAC | |
| ATP5B | F | 9 | TGTCGATCTGCTAGCTCCCTA | ATP synthase, H+ transporting, mitochondrial F1 complex, beta polypeptide |
| | R | 10 | TCAGTACAGTCTTGCCAACTCC | |
| CAPN6 | F | 11 | TGGAAAGGTGGTGTGGAAAC | calpain 6 |
| | R | 12 | GTCAGCTGGTGGTTGCTAA | |
| CIR1 | F | 13 | TCAATGCAAGTTCGGTTCCC | corepressor interacting with RBPJ, 1 |
| | R | 14 | CAAACCCACTGTTTCTCATCTCC | |
| CMTM5 | F | 15 | AAGGGCATCCTGCTGGAAA | CKLF-like MARVEL transmembrane domain containing 5 |
| | R | 16 | CAGAGATGGAGGCCGTGAA | |
| CTNNA2 | F | 17 | TGGAAATCCGGACGCTAACA | catenin (cadherin-associated protein), alpha 2 |
| | R | 18 | GCCTTTGTTGCTTGTGTTGAC | |
| CXCL1 | F | 19 | CTTGCCTCAATCCTGCATCC | chemokine (C-X-C motif) ligand 1 (melanoma growth stimulating activity, alpha) |
| | R | 20 | AGCCACCAGTGAGCTTCC | |
| CXCL6 | F | 21 | ACGCTGAGAGTAAACCCCAAA | chemokine (C-X-C motif) ligand 6 |
| | R | 22 | CAAACTTGCTTCCCGTTCTTCA | |
| FOXP3 | F | 23 | TGTGGGGTAGCCATGGAAA | forkhead box P3 |
| | R | 24 | GGGTCGCATGTTGTGGAA | |
| GPX3 | F | 25 | TTTGTCAACGTGGCCAGCTA | glutathione peroxidase 3 (plasma) |
| | R | 26 | AAAGCCCAGAATGACCAGACC | |
| HBA1 | F | 27 | TCTCCTGCCGACAAGACCAA | hemoglobin, alpha 1 |
| | R | 28 | GTGGTGGGGAAGGACAGGA | |
| HBG1 | F | 29 | GCAAGAAGGTGCTGACTTCC | hemoglobin, gamma A |
| | R | 30 | GCTTGTCACAGTGCAGTTCA | |
| HMBS | F | 31 | AGAGTGATTCGCGTGGGTA | hydroxymethylbilane synthase |
| | R | 32 | CTTTCAATGTTGCCACCACAC | |
| HOXB7 | F | 33 | TTCCGGATCTACCCCTGGAT | homeobox B7 |
| | R | 34 | TCTGGTAGCGGGTGTAGGT | |
| IL1R1 | F | 35 | GGGCAAGCAATATCCTATTACCC | interleukin 1 receptor, type I |
| | R | 36 | TAGCTGGGCTCACAATCACA | |
| IL4 | F | 37 | CAGCTGATCCGATTCCTGAAA | interleukin 4 |
| | R | 38 | GTTGGCTTCCTTCACAGGAC | |
| IL5 | F | 39 | ACTCTGAGGATTCCTGTTCCTGTA | interleukin 5 (colony-stimulating factor, eosinophil) |
| | R | 40 | CCAGTGTGCCTATTCCCTGAAA | |
| ITGB1 | F | 41 | GAATGTATACAAGCAGGGCCAAA | integrin, beta 1 (fibronectin receptor, beta polypeptide, antigen CD29 includes MDF2, MSK12) |
| | R | 42 | CGTGCAGAAGTAGGCATTCC | |
| ITGA9 | F | 43 | AAATGCAGCGTGGGATTTCC | integrin, alpha 9 |
| | R | 44 | CCAGACAGGTGGCTTGTATCA | |
| MAOA | F | 45 | ATGGTCTCGGGAAGGTGAC | monoamine oxidase A |
| | R | 46 | GGTGATTTCTACCGCTGGAAC | |
| MAPK1 | F | 47 | TTGGTACAGGGCTCCAGAAA | mitogen-activated protein kinase 1 |
| | R | 48 | TCTGCCAGAATGCAGCCTA | |
| MMP9 | F | 49 | AGTGGCACCACCACAACA | matrix metallopeptidase 9 (gelatinase B, 92kDa gelatinase, 92kDa type IV collagenase) |
| | R | 50 | GCAAAGGCGTCGTCAATCA | |
| MT1F | F | 51 | TCCTCGGCTTGCAATGGA | metallothionein 1F |
| | R | 52 | CTTTGCACTTGCAGGAACCA | |
| MT1H | F | 53 | GCTCCTGCAAGTGCAAAA | metallothionein 1H |
| | R | 54 | GCAGCTGCACTTCTCTGAC | |
| MT1L | F | 55 | CAGCTCCTGCAAGTGCAAA | metallothionein 1 L (gene/pseudogene) |
| | R | 56 | GACGCCCCTTTGCAGAC | |
| MT1X | F | 57 | CCTCGAAATGGACCCCAACT | metallothionein 1X |
| | R | 58 | GCACTCTTTGCATTTGCAGGA | |
| NFKBIA | F | 59 | CACCTCCACTCCATCCTGAA | nuclear factor of kappa light polypeptide gene enhancer in B-cells inhibitor, alpha |
| | R | 60 | GGTAGCCATGGATAGAGGCTAA | |
| PGR | F | 61 | AGCCAAGAAGAGTTCCTCTGTA | progesterone receptor |
| | R | 62 | TTGACTTCGTAGCCCTTCCA | |
| PGRMC1 | F | 63 | GGCAAGGTGTTCGATGTGAC | progesterone receptor membrane component 1 |
| | R | 64 | CTCTTCCAGCAAAGACCCCATA | |
| PLA2G4A | F | 65 | AGGCTCCACAATGGAGGAA | phospholipase A2, group IVA (cytosolic, calcium-dependent) |
| | R | 66 | TCATCATCACTGTCCGAGCTA | |
| PTGS1 | F | 67 | GCGGCTCTTTAAGGATGGGAA | prostaglandin-endoperoxide synthase 1 (prostaglandin G/H synthase and cyclooxygenase) |
| | R | 68 | AACACAGGCGCCTCTTCTAC | |
| RHOA | F | 69 | GTGCCCACAGTGTTTGAGAA | ras homolog family member A |
| | R | 70 | TGTGTCCCACAAAGCCAAC | |
| RPL13A | F | 71 | GAGGCCCCTACCACTTCC | ribosomal protein L13a |
| | R | 72 | GCCGTCAAACACCTTGAGAC | |
| SCGB2A2 | F | 73 | CTCTCCCAGCACTGCTAC | secretoglobin, family 2A, member 2 |
| | R | 74 | CTTGTGGATTGATTGTCTTGGAA | |
| SDHA | F | 75 | ACATCGGAACTGCGACTCA | succinate dehydrogenase complex, subunit A, flavoprotein (Fp) |
| | R | 76 | TTCTTGCAACACGCTTCCC | |
| SERPINA1 | F | 77 | CCACGATATCATCACCAAGTTCC | serpin peptidase inhibitor, clade A (alpha-1 antiproteinase, antitrypsin), member 1 |
| | R | 78 | TTGACCCAGGACGCTCTTCA | |
| TFF3 | F | 79 | GGCCTTGCTGTCCTCCA | trefoil factor 3 (intestinal) |
| | R | 80 | CCCGGTTGTTGCACTCC | |
| ACTB | F | 81 | CCAACCGCGAGAAGATGAC | actin, beta |
| | R | 82 | TAGCACAGCCTGGATAGCAA | |
| B2M | F | 83 | TTAGCTGTGCTCGCGCTAC | beta-2-microglobulin |
| | R | 84 | CTCTGCTGGATGACGTGAGTAA | |
| CYC1 | F | 85 | CCATGGCCCCTCCCATCTA | cytochrome c-1 |
| | R | 86 | GGTGCACACATCCTTGGCTA | |
| EMG1 | F | 87 | CAGACCCGAATTCCCAGAAC | EMG1 N1-specific pseudouridine methyltransferase |
| | R | 88 | CTCGAACACTGAGCTTGTGTAA | |
| GAPDH | F | 89 | GAACGGGAAGCTTGTCATCAA | glyceraldehyde-3-phosphate dehydrogenase |
| | R | 90 | A TCGCCCCACTTGATTTTGG | |
| TBP | F | 91 | TGCCCGAAACGCCGAATATA | TATA box binding protein |
| | R | 92 | CGTGGTTCGTGGCTCTCTTA | |
| TOP1 | F | 93 | ACGCTACAGCAGCAGCTAA | topoisomerase (DNA) I |
| | R | 94 | AGCTCGATTGGCACGGTTA | |
| YWHAZ | F | 95 | AGACAGCACGCTAATAATGCA | tyrosine 3-monooxygenase/tryptophan 5-monooxygenase activation protein, zeta |
| | R | 96 | AGCTTCGTCTCCTTGGGTA | |

## Claims

1. A kit for determining the receptivity status of an endometrium comprising means for quantifying the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, wherein said kit comprises means for quantifying the expression of up to 100 genes, preferably up to 48 genes.

2. The kit according to claim 1, further comprising means for quantifying the expression of at least one of the following genes: ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

3. The kit according to any one of claims 1 or 2, comprising means for quantifying the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

4. The kit according to any one of claims 1 to 3, wherein the means for quantifying the genes comprise a pair of primers for each one of the genes.

5. The kit according to any one of claims 1 to 4, further comprising a pair of primers for at least one reference gene, or further comprising a pair of primers for at least on reference gene, wherein the reference gene or genes is or are selected from actin, beta-2 microglobulin, cytochrome C1, EMG1 N1-specific pseudouridine methyltransferase, glyceraldehyde-3-phosphate dehydrogenase, TATA-box binding protein, topoisomerase (DNA) I, tyrosine 3-monooxygenase/tryptophan 5-monooxygenase activation protein zeta and combinations thereof.

6. The kit according to any one of claims 1 to 5, further comprising a specific probe for each one of the genes.

7. The kit according to claim 6, wherein the probe is labeled, preferably dual-labeled.

8. The kit according to any one of claims 1 to 7, further comprising at least one of the following: a DNA polymerase, a nucleotide mix, a buffer, DNase-free water.

9. A method for determining the receptivity status of an endometrium comprising the quantification of the expression of the following genes: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, in a sample.

10. The method according to claim 9, further comprising the quantification of the expression of at least one of the following genes: ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

11. The method according to any one of claims 9 or 10, wherein the expression of the following genes is quantified: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

12. The method according to any one of claims 9 to 11, further comprising quantifying the expression of at least one reference gene in the sample.

13. The method according to any one of claims 9 to 12, wherein the quantification is made by qPCR, preferably by RT-qPCR.

14. The method according to any one of claims 9 to 13, wherein the sample is a cDNA sample, preferably a cDNA obtained from an RNA sample from a female subject, preferably a female human.

## Patentansprüche

1. Kit zur Bestimmung des Rezeptivitätsstatus eines Endometriums, umfassend Mittel zur Quantifizierung der Expression der folgenden Gene: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, wobei das Kit Mittel zur Quantifizierung der Expression von bis zu 100 Genen, vorzugsweise bis zu 48 Genen, umfasst.

2. Kit nach Anspruch 1, ferner umfassend Mittel zur Quantifizierung der Expression von mindestens einem der folgenden Gene: ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

3. Kit nach einem der Ansprüche 1 oder 2, umfassend Mittel zur Quantifizierung der Expression der folgenden Gene: AREG, ATP5B, CAPN6, CIR1, CMTM5, CTNNA2,CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

4. Kit nach einem der Ansprüche 1 bis 3, wobei die Mittel zur Quantifizierung der Gene ein Paar von Primern für jedes der Gene umfassen.

5. Kit nach einem der Ansprüche 1 bis 4, ferner umfassend ein Paar von Primern für mindestens ein Referenzgen, oder ferner umfassend ein Paar von Primern für mindestens ein Referenzgen, wobei das Referenzgen oder die Referenzgene ausgewählt sind aus Actin, beta-2-Mikroglobulin, Cytochrom C1, EMG1 N1-spezifische Pseudouridin-Methyltransferase, Glyceraldehyd-3-Phosphat-Dehydrogenase, TATA-Box-Bindungsprotein, Topoisomerase (DNA) I, Tyrosin-3-Monooxygenase/Tryptophan 5-Monooxygenase-Aktivierungsprotein Zeta und Kombinationen davon.

6. Kit nach einem der Ansprüche 1 bis 5, ferner umfassend eine spezifische Sonde für jedes der Gene.

7. Kit nach Anspruch 6, wobei die Sonde beschriftet ist, vorzugsweise doppelt beschriftet.

8. Kit nach einem der Ansprüche 1 bis 7, ferner umfassend mindestens eines von Folgendem: eine DNA-Polymerase, eine Nukleotidmischung, einen Puffer, DNase-freies Wasser.

9. Methode zur Bestimmung des Rezeptivitätsstatus eines Endometriums, umfassend die Quantifizierung der Expression der folgenden Gene: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, in einer Probe.

10. Methode nach Anspruch 9, ferner umfassend die Quantifizierung der Expression von mindestens einem der folgenden Gene: ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

11. Methode nach einem der Ansprüche 9 oder 10, wobei die Expression der folgenden Gene quantifiziert wird: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

12. Methode nach einem der Ansprüche 9 bis 11, ferner umfassend die Quantifizierung der Expression von mindestens einem Referenzgen in der Probe.

13. Methode nach einem der Ansprüche 9 bis 12, wobei die Quantifizierung durch qPCR, vorzugsweise durch RT-qPCR, durchgeführt wird.

14. Methode nach einem der Ansprüche 9 bis 13, wobei die Probe eine cDNA-Probe ist, vorzugsweise eine cDNA, die aus einer RNA-Probe von einem weiblichen Subjekt, vorzugsweise einem weiblichen Menschen, erhalten wurde.

## Revendications

1. Un kit pour déterminer l'état de réceptivité d'un endomètre comprenant des moyens pour quantifier l'expression des gènes suivants: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, dans lequel ledit kit comprend : des moyens pour quantifier l'expression de jusqu'à 100 gènes, de préférence jusqu'à 48 gènes.

2. Le kit selon la revendication 1, comprenant en outre des moyens pour quantifier l'expression d'au moins l'un des gènes suivants : ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

3. Le kit selon l'une des revendications 1 ou 2, comprenant des moyens pour quantifier l'expression des gènes suivants: AREG, ATP5B, CAPN6, CIR1, CMTM5, CTNNA2,CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

4. Le kit selon l'une des revendications 1 à 3, dans lequel les moyens pour quantifier les gènes comprennent une paire d'amorces pour chacun des gènes.

5. Le kit selon l'une des revendications 1 à 4, comprenant en outre une paire d'amorces pour au moins un gène de référence, ou comprenant en outre une paire d'amorces pour au moins un gène de référence, dans lequel le ou les gènes de référence sont choisis parmi l'actine, la microglobuline bêta 2, la cytochrome C1, la méthyltransférase pseudouridine spécifique à EMG1 N1, la glycéraldéhyde-3-phosphate déshydrogénase, la protéine de liaison à la boîte TATA, la topoisomérase (ADN) I, la protéine d'activation zêta de la tyrosine 3-monooxygénase/tryptophane 5-monooxygénase et des combinaisons de celles-ci.

6. Le kit selon l'une des revendications 1 à 5, comprenant en outre une sonde spécifique pour chacun des gènes.

7. Le kit selon la revendication 6, dans lequel la sonde est étiquetée, de préférence doublement étiquetée.

8. Le kit selon l'une des revendications 1 à 7, comprenant en outre au moins l'un des éléments suivants: une ADN polymérase, un mélange de nucléotides, un tampon, de l'eau exempte de DNase.

9. Un procédé de détermination de l'état de réceptivité d'un endomètre comprenant la quantification de l'expression des gènes suivants: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, dans un échantillon.

10. Le procédé selon la revendication 9, comprenant en outre la quantification de l'expression d'au moins l'un des gènes suivants: ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

11. Le procédé selon l'une des revendications 9 ou 10, dans lequel l'expression des gènes suivants est quantifiée: AREG, ATP5B, CAPN6, CIR1, CMTM5, CTNNA2, CXCL6, FOXP3, HBA1, HBG1, HMBS, HOXB7, IL1R1, IL4, IL5, ITGB1, MAPK1, NFKBIA, PGRMC1, PLA2G4A, RHOA, SDHA, SERPIN1, ANXA4, AQP3, ARG2, CXCL1, GPX3, ITGA9, MAOA, MMP9, MT1F, MT1H, MT1L, MT1X, PGR, PTGS1, RPL13A, SCGB2A2, TFF3.

12. Le procédé selon l'une des revendications 9 à 11, comprenant en outre la quantification de l'expression d'au moins un gène de référence dans l'échantillon.

13. Le procédé selon l'une des revendications 9 à 12, dans lequel la quantification est effectuée par qPCR, de préférence par RT-qPCR.

14. Le procédé selon l'une des revendications 9 à 13, dans lequel l'échantillon est un échantillon d'ADNc, de préférence un ADNc obtenu à partir d'un échantillon d'ARN d'un sujet féminin, de préférence un être humain de sexe féminin.
